# FASCICULE DE BREVET EUROPEEN

(11) **EP 3 519 030 B1**
(45) Date de publication et mention de la délivrance du brevet: **27.07.2022**
(21) Numéro de dépôt: 17787223.1
(22) Date de dépôt: 27.09.2017
(51) Int. Cl.: A61M 21/02, A61M 21/00

(54) **DISPOSITIF D'AIDE A L'ENDORMISSEMENT**
SCHLAFHILFSVORRICHTUNG
SLEEP AID DEVICE

(30) Priorité: 28.09.2016 FR 1659221
(43) Date de publication de la demande: 07.08.2019
(73) Titulaire: Helight, 42100 Saint-Etienne (FR)
(72) Inventeur: GRANGE, Jérôme, Armand, Joseph, 42740 Saint-Paul-enJarez (FR); MONCORGER, Jean, Marc, 69005 Lyon (FR); THEVENET, Julien, Michel, 38510 Saint Sorlin de Morestel (FR); FELIX, Didier, Denis, 38630 Corbelin (FR); GRONFIER, Claude, 69003 Lyon (FR)
(74) Mandataire: Weber, Jean-François
(86) Numéro de dépôt international: PCT/FR2017/052621
(87) Numéro de publication internationale: WO 2018/060616

(56) Documents cités:
- WO-A1-2012/137159
- DE-A1-102012 004 647
- US-A1- 2003 231 495

## Description

### DOMAINE TECHNIQUE

La présente invention concerne le domaine général de l'aide à l'endormissement et tout particulièrement des dispositifs d'aide à l'endormissement qui émettent de la lumière. Plus particulièrement, l'invention concerne un dispositif d'aide à l'endormissement d'un utilisateur, comprenant une source lumineuse conçue pour émettre une lumière sensiblement monochromatique rouge ou quasi monochromatique favorisant l'endormissement de l'utilisateur.

L'invention concerne également un système d'aide à l'endormissement d'un utilisateur comprenant un dispositif d'aide à l'endormissement.

La présente description montre également un procédé d'endormissement d'un utilisateur dans lequel on soumet un utilisateur à une lumière monochromatique rouge favorisant l'endormissement de l'utilisateur à partir d'une source lumineuse.

### TECHNIQUE ANTERIEURE

On sait que la lumière, et notamment la lumière blanche, a une grande influence sur les rythmes circadiens de l'homme et en particulier sur son sommeil. En effet, le rythme naturel du sommeil de l'homme est normalement sensiblement calqué sur celui du soleil de manière à être éveillé durant la journée et à dormir durant la nuit.

Plus précisément, on sait que le sommeil chez l'homme, et par conséquent le bon déroulement de son rythme circadien, est notamment dépendant de la glande pinéale (ou épiphyse) et de l'hormone qu'elle sécrète, la mélatonine. En effet, cette hormone est connue pour réguler les rythmes biologiques de l'homme, y compris son sommeil. En particulier, on sait que certaines composantes de la lumière visible par l'homme (c'est-à-dire de la lumière blanche) sont à même de dérégler le bon fonctionnement de cette glande et notamment de bloquer la production de mélatonine, comme par exemple la lumière bleue. Inversement, il est connu que la lumière rouge n'a pas ou très peu d'influence sur la production de cette hormone, et est par conséquent pratiquement sans influence sur le rythme circadien de l'homme.

Afin de ne pas perturber le rythme circadien de l'homme, il a été proposé des dispositifs d'éclairage émettant une lumière rouge qui n'a pas ou peu d'influence sur la production de mélatonine et par conséquent sur le bon déroulement du sommeil de l'homme. Il est en particulier connu d'utiliser de tels dispositifs dans des lieux destinés au sommeil mais où il est toutefois nécessaire de garantir un certain niveau d'éclairage, notamment pour permettre un déplacement en toute sécurité, sans pour autant trop perturber le sommeil.

Ainsi, il est par exemple connu d'utiliser de tels dispositifs d'éclairage dans un cadre domestique, au sein d'une habitation, par exemple dans une chambre, un couloir, des escaliers, ou même dans un réfrigérateur. En effet, lorsqu'un résident de l'habitation ainsi équipée se réveille au cours de la nuit et souhaite se déplacer, il pourra utiliser l'éclairage fourni par les dispositifs connus pour se déplacer en toute sécurité sans avoir à utiliser l'éclairage traditionnel de son logement. Ainsi, l'utilisateur est préservé de la lumière blanche (et de ses composantes, notamment bleues, nocives pour son rythme circadien) et peut ainsi retrouver le sommeil plus facilement.

Ces dispositifs connus sont souvent équipés de capteurs de mouvement pour détecter le déplacement de l'utilisateur et ainsi s'allumer sur son passage puis s'éteindre automatiquement lorsque l'utilisateur n'est plus en mouvement. Afin d'optimiser la consommation d'énergie de ces dispositifs connus, ces derniers peuvent également être équipés de capteurs de lumière, afin de n'entrer en fonction que lorsqu'il fait totalement noir.

Ces dispositifs connus donnent généralement satisfaction à leurs utilisateurs en sécurisant leurs éventuels déplacements nocturnes tout en limitant l'impact sur leur ré-endormissement.

Ces dispositifs connus sont toutefois limités à cette fonction duale de sécurisation des déplacements et de préservation du ré-endormissement, et ne sont pas adaptés pour favoriser l'endormissement initial proprement dit, c'est-à-dire pour contribuer directement et efficacement, de façon positive, au passage de l'état éveillé à l'état de sommeil inconscient, en particulier au début de la phase de sommeil nocturne.

Au contraire, ces dispositifs ne doivent pas être placés dans une chambre à coucher par exemple, car ils pourraient perturber l'endormissement initial en soumettant l'utilisateur à des cycles d'allumage/extinction intempestifs, commandés accidentellement par d'éventuels mouvements (volontaires ou inconscients) de l'utilisateur dans son sommeil, susceptibles de déclencher les capteurs de mouvement équipant les dispositifs d'éclairage connus en question.

Le document WO-2012/137159A1 décrit le préambule de la revendication 1.

### EXPOSE DE L'INVENTION

L'invention est définie par le jeu de revendications ci-joint.

Les objets assignés à la présente invention visent en conséquence à remédier aux différents inconvénients énumérés précédemment et à proposer de nouveaux dispositif et système d'aide à l'endormissement d'un utilisateur qui, tout en étant particulièrement efficaces pour aider à l'endormissement d'un utilisateur, sont de conceptions extrêmement simples et bon marché.

Un autre objet de l'invention vise à proposer de nouveaux dispositif et système d'aide à l'endormissement qui permettent de contribuer efficacement à l'endormissement de chaque utilisateur en s'adaptant à ses caractéristiques d'endormissement.

Un autre objet de l'invention vise à proposer de nouveaux dispositif et système d'aide à l'endormissement qui permette de contribuer au bon règlement du rythme circadien du sommeil de l'homme.

Un autre objet de l'invention vise à proposer de nouveaux dispositif et système d'aide à l'endormissement qui vise à ne pas perturber la production de mélatonine.

Un autre objet de l'invention vise à proposer de nouveaux dispositif et système d'aide à l'endormissement qui soit particulièrement économe en énergie.

Un autre objet de l'invention vise à proposer de nouveaux dispositif et système d'aide à l'endormissement qui soit bénéfique pour l'organisme même dans un environnement qui n'est pas totalement obscur.

Un autre objet de l'invention vise à proposer de nouveaux dispositif et système d'aide à l'endormissement qui puisse être utilisé dans une pluralité de lieux différents.

Un autre objet de l'invention vise à proposer de nouveaux dispositif et système d'aide à l'endormissement qui soit particulièrement simple à utiliser.

Un autre objet de l'invention vise à proposer de nouveaux dispositif et système d'aide à l'endormissement qui soit particulièrement intuitif, ergonomique et ludique à utiliser.

Un autre objet de l'invention vise à proposer de nouveaux dispositif et système d'aide à l'endormissement qui soit particulièrement compact, robuste et fiable.

Un autre objet de l'invention vise à proposer de nouveaux dispositif et système d'aide à l'endormissement qui soit facilement transportable.

Un autre objet de l'invention vise à proposer de nouveaux dispositif et système d'aide à l'endormissement d'un utilisateur qui soit particulièrement efficace et efficient.

Un autre objet de l'invention vise à proposer de nouveaux dispositif et système d'aide à l'endormissement d'un utilisateur qui soit particulièrement simple, intuitif, ergonomique et ludique à utiliser.

Un autre objet de l'invention vise à proposer un nouveau procédé d'endormissement qui soit particulièrement efficace et efficient.

Un autre objet de l'invention vise à proposer un nouveau procédé d'endormissement qui soit adapté à une pluralité d'utilisateurs.

Un objet de la description vise à proposer un nouveau procédé d'endormissement qui permette de contribuer au bon règlement du rythme circadien du sommeil de l'homme.

Un autre objet de la description vise à proposer un nouveau procédé d'endormissement qui vise à ne pas perturber la production de mélatonine.

Les objets assignés à l'invention sont atteints à l'aide d'un dispositif d'aide à l'endormissement conforme à l'objet de la revendication 1.

Les objets assignés à l'invention sont également atteints à l'aide d'un système d'aide à l'endormissement conforme à l'objet de la revendication 13.

### DESCRIPTIF SOMMAIRE DES DESSINS

D'autres particularités et avantages de l'invention apparaîtront et ressortiront plus en détails à la lecture de la description faite ci-après, en référence aux dessins annexés, donnés uniquement à titre d'exemple illustratif et non limitatif, dans lesquels :
- La figure 1 est une vue en perspective du dessus d'un dispositif d'aide à l'endormissement selon un mode de réalisation préférentiel de l'invention.
- La figure 2 est une vue en perspective du dessous du dispositif d'aide à l'endormissement de la figure 1.
- La figure 3 est une vue de dessous du dispositif d'aide à l'endormissement de la figure 1.
- La figure 4 est une vue de côté du dispositif d'aide à l'endormissement de la figure 1.
- La figure 5 est une vue de dessus du dispositif d'aide à l'endormissement de la figure 1.
- La figure 6 est une vue en coupe du dispositif d'aide à l'endormissement de la figure 4.
- La figure 7 est une vue de dessus du diffuseur équipant le dispositif d'aide à l'endormissement de la figure 1.
- La figure 8 est une vue de côté du diffuseur de la figure 7.
- La figure 9 est une représentation schématique d'un système tel que décrit par l'invention, ledit système comprenant notamment le dispositif de la figure 1.
- Les figures 10 à 13 représentent chacune un exemple particulier de loi prédéfinie qui régit le fonctionnement du dispositif de la figure 1.

### MEILLEURE MANIERE DE REALISER L'INVENTION

L'invention concerne en tant que tel un dispositif d'aide à l'endormissement d'un utilisateur. Ledit dispositif comprend préférentiellement un appareil, un moyen, un outil, une machine, un instrument, etc. qui est avantageusement conçu pour aider un utilisateur à s'endormir, ou tout du moins à se reposer, en conférant avantageusement à la pièce dans laquelle se trouve l'utilisateur un éclairage particulièrement propice à l'endormissement et / ou au repos. Préférentiellement ledit éclairage est un éclairage visible pour un être humain et avantageusement un éclairage qui lui apparait rouge, comme cela sera détaillé par la suite.

De manière préférentielle, l'utilisateur de l'appareil est un être humain, ce dernier étant de n'importe quel âge et de n'importe quel sexe. Par extension et sans sortir du cadre de l'invention, l'utilisateur peut également désigner un animal dont au moins certaines propriétés corporelles, et en particuliers certaines caractéristiques cutanées et / ou optiques se rapprochent sensiblement de celles des êtres humains.

De préférence, ledit dispositif est un dispositif de photomodulation ou photobiomodulation, c'est-à-dire un dispositif capable d'émettre une lumière particulière pour différentes indications, et notamment pour favoriser l'endormissement et / ou le repos, comme cela sera détaillé par la suite. Ledit dispositif est alors avantageusement destiné à interagir, par l'intermédiaire de la lumière qu'il émet, avec les récepteurs cellulaires de lumière situés dans l'œil ou au niveau de la peau de l'utilisateur.

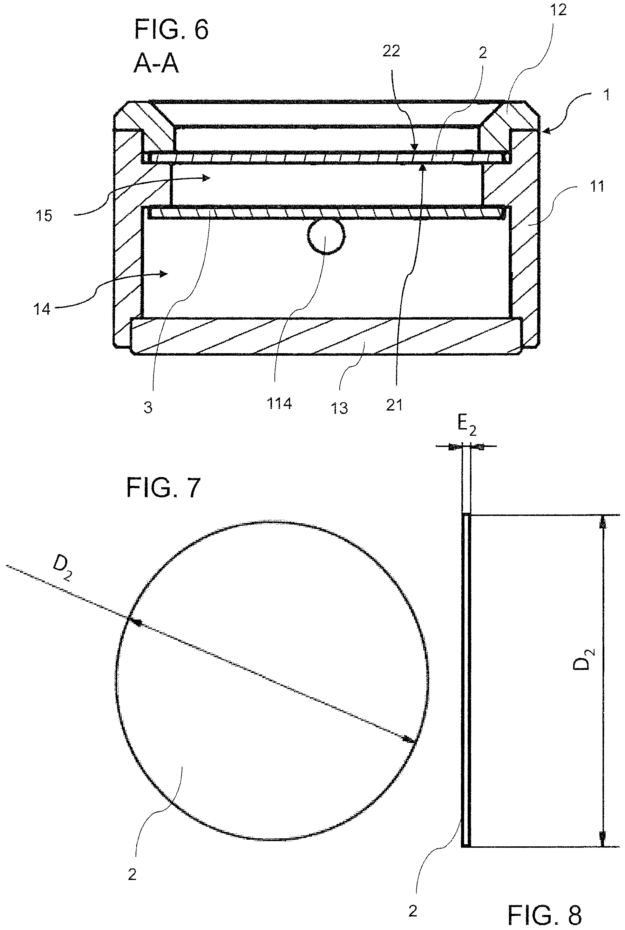

Selon l'invention, ledit dispositif comprend une source lumineuse 3 ou éventuellement une pluralité de sources lumineuses 3, comme on peut le voir par exemple sur la figure 6. Ladite source lumineuse 3 est avantageusement formée par n'importe quelle source de lumière, c'est-à-dire n'importe quel élément, moyen, dispositif ou ensemble de composants conçu pour émettre de la lumière, comme par exemple une ampoule, et par exemple une ampoule à incandescence, une ampoule fluorescente, une ampoule halogène, ou avantageusement une ampoule LED (c'est-à-dire une ampoule composée d'une ou de plusieurs diodes électroluminescentes).

Selon le mode de réalisation préférentiel illustré par les figures, ladite source de lumière comprend au moins une diode électroluminescente LED, et préférentiellement une pluralité de LED, et se présente préférentiellement sous la forme d'une plaque de LED ou d'un module LED, ce dernier pouvant avantageusement prendre la forme d'un disque, comme cela est bien connu en tant que tel. En d'autres termes, et comme cela est par exemple visible sur la figure 6, ladite source lumineuse 3 comprend un module LED préférentiellement sous forme de disque, ledit module étant composé d'un réseau de diodes électroluminescentes LED préférentiellement conçues pour s'éclairer simultanément de manière à produire une intensité lumineuse supérieure à celle qui serait produite par une seule LED. II est ainsi possible d'avoir une source lumineuse 3 particulièrement compacte et puissante.

Selon l'invention, ladite source lumineuse 3 est conçue pour émettre une lumière sensiblement monochromatique rouge (ou quasi-monochromatique) favorisant l'endormissement de l'utilisateur. En effet, comme cela a été précisé précédemment, il est connu que certaines composantes de la lumière visibles par l'homme, et en particulier la lumière rouge, n'ont pas ou peu d'influence sur la production de mélatonine, une des hormones naturelles présente dans le corps humain régulant les rythmes circadiens de l'homme. En outre, une telle lumière rouge est remarquablement connue pour ne présenter aucune toxicité pour l'organisme, y compris pour la rétine, à l'inverse des rayons ultra-violets par exemple.

Ainsi, ladite source lumineuse 3 émet une lumière appartenant au spectre de la lumière visible, c'est-à-dire une lumière dont la longueur d'onde est sensiblement comprise entre 400 nm et 700 nm.

Selon l'invention, ledit dispositif est avantageusement conçu pour émettre une lumière sensiblement monochromatique, ou quasi-monochromatique, voire monochromatique, c'est-à-dire que c'est une lumière qui ne comprend avantageusement qu'une seule longueur d'onde, ou du moins qui tend à ne comporter qu'une seule longueur d'onde, moyennant la précision inhérente aux différents composants mis en œuvre. Avantageusement, ladite longueur d'onde est préférentiellement comprise entre 600 nm et 670 nm et avantageusement entre 615 nm et 640 nm, ces plages de longueur d'onde correspondant à une lumière rouge, cette dernière présentant l'avantage remarquable d'apaiser l'utilisateur, de le relaxer, et de l'aider à trouver le sommeil.

De manière avantageuse, ladite lumière émise par ladite source lumineuse 3 est une lumière rouge dont la longueur d'onde est de préférence égale à 625 nm + ou - 15 nm.

Avantageusement, la lumière émise par ledit dispositif est une lumière froide, au rayonnement non ionisant, émise préférentiellement par des LED de faible intensité (inférieure à 190 mW / cm²). Plus particulièrement, les caractéristiques techniques
des LED sont avantageusement choisies pour que les LED puissent transférer de l'énergie vers les cellules et / ou apporter des signaux aux récepteurs cellulaires situés dans l'œil ou au niveau de la peau de l'utilisateur.

Selon l'invention, ledit dispositif comprend une unité de commande conçue pour contrôler automatiquement ladite source lumineuse 3. A cette fin, ladite unité de commande comprend avantageusement un ou plusieurs éléments, moyens, outils ou dispositifs de contrôle automatique, comme par exemple et de manière non limitative un calculateur, un capteur, une minuterie, un processeur, une mémoire, etc. En d'autres termes, ladite source lumineuse 3 est dépendante de ladite unité de commande, de sorte que cette dernière est notamment capable de contrôler, de définir, de régler, de vérifier etc. les propriétés physiques de ladite source lumineuse 3, et notamment sa puissance P, et ce sans intervention de l'utilisateur, ni même l'intervention d'une personne quelconque pendant le fonctionnement dudit dispositif.

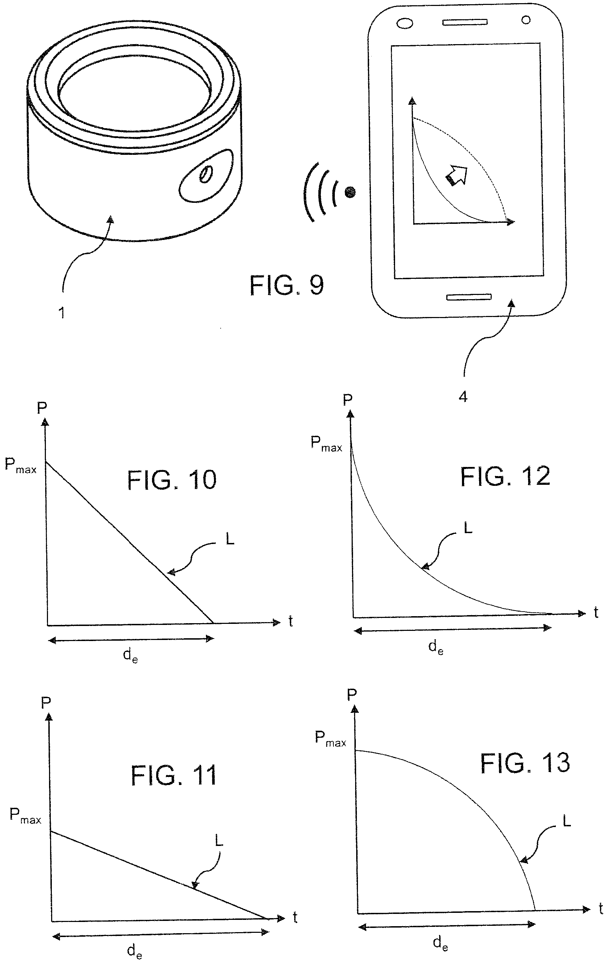

Toujours selon l'invention, ladite source lumineuse 3 est contrôlée automatiquement conformément à une loi prédéfinie L, c'est-à-dire selon un modèle, un cycle ou un profil établi à l'avance, par exemple par les concepteurs dudit dispositif. Préférentiellement, ladite loi L est avantageusement enregistrée dans une mémoire de ladite unité de commande, par exemple lors de la fabrication dudit dispositif. Ladite loi est alors une loi selon laquelle la puissance lumineuse P émise par ladite source lumineuse 3 est progressivement décroissante au cours du temps t. En d'autres termes, ladite loi prédéfinie L régit le fonctionnement que ladite source lumineuse 3 doit suivre en contrôlant avantageusement au moins la puissance de cette dernière, et plus précisément en faisant diminuer de manière progressive et continue ladite puissance P au cours du temps. Avantageusement, ladite loi prédéfinie L peut être matérialisée par une fonction mathématique définissant la puissance P de ladite source lumineuse 3 en fonction du temps t, comme l'illustrent par exemple les figures 10 à 13 qui seront détaillées par la suite. Ladite puissance P décroit alors automatiquement et progressivement en fonction du temps t, de sorte que la puissance lumineuse émise par ladite source lumineuse 3 à un instant T est toujours supérieure à la puissance lumineuse émise à un instant ultérieur T+1. En d'autres termes, plus le temps s'écoule, plus la puissance émise par ledit dispositif diminue. Ainsi, grâce à ladite loi prédéfinie L, ledit dispositif fonctionne selon un cycle automatique prédéfini, ce dernier étant avantageusement un cycle de décroissance automatique de puissance.

Selon l'invention, la puissance P émise est alors contrôlée par ladite loi prédéfinie L de manière à évoluer automatiquement entre une valeur de puissance maximale Pₘₐₓ et une valeur de puissance minimale, comme on peut par exemple le voir sur les figures 10 à 13. Préférentiellement, ladite valeur de puissance minimale est une valeur sensiblement nulle. En d'autres termes, ladite loi prédéfinie L est telle qu'elle va faire automatiquement passer ladite puissance P de ladite source lumineuse 3 d'une valeur de puissance maximale Pₘₐₓ à une puissance sensiblement nulle, c'est-à-dire à une puissance proche de zéro voire une puissance égale à zéro, et ce préférentiellement selon un mode de décroissance prédéfini et automatique, c'est-à-dire prévu à l'avance et sans intervention de l'utilisateur. Avantageusement, lorsque ledit dispositif atteint ladite puissance sensiblement nulle, ce dernier n'émet plus de lumière. Ainsi, ledit dispositif est conçu pour faire varier ladite puissance P émise par ladite source lumineuse selon un profil de décroissance particulier défini à l'avance.

Préférentiellement ledit dispositif comprend une première interface utilisateur permettant à l'utilisateur d'interagir avec ledit dispositif. Ladite première interface utilisateur est alors au moins conçue pour permettre à l'utilisateur de mettre en fonction (mettre en marche) ledit dispositif et comprend par exemple un bouton de mise en marche, par exemple un bouton poussoir préférentiellement monostable. En d'autres termes, dès que l'utilisateur appuie sur ledit bouton de mise en marche, ledit dispositif entre préférentiellement en fonctionnement selon ladite loi prédéfinie L. Toutefois, il est parfaitement envisageable, et ce sans sortir du cadre de l'invention, que ledit bouton puisse être utilisé pour d'autres fonctions, notamment à partir d'un appui long sur ce dernier, comme par exemple pour l'extinction prématurée dudit dispositif (c'est-à-dire son extinction avant la fin de ladite loi prédéterminée L) ou bien encore pour le déclanchement d'une minuterie par exemple, comme cela sera détaillé par la suite.

Préférentiellement, ledit dispositif a une puissance de sortie maximale Pₘₐₓ sensiblement égale à 40 mW/cm². En d'autres termes, lorsque ledit dispositif est en fonctionnement, la puissance émise par ledit dispositif, et plus précisément par ladite source lumineuse 3, est inférieure ou égale à 40 mW/cm², de manière à ce que, une fois le dispositif posé sur une table de nuit par exemple, en condition normale d'utilisation, par exemple à une distance moyenne de 50 cm de l'utilisateur en position allongée en phase de pré-endormissement, les récepteurs de type mélanospine situés dans la rétine ne soient pas exposés à une lumière de plus de 400 µW/cm², pour ladite longueur d'onde, si le regard de l'utilisateur était dirigé vers la source lumineuse, cette puissance correspondant avantageusement à une puissance seuil acceptable par l'organisme humain en phase d'endormissement et / ou de repos.

De manière avantageuse, ladite valeur de puissance lumineuse maximale Pₘₐₓ est supérieure à 600 lux et est avantageusement sensiblement égale à 885 lux. En d'autres termes, lorsque ledit dispositif est en fonctionnement, la puissance lumineuse émise par ledit dispositif, est inférieure ou égale à 885 lux, cette puissance correspondant avantageusement à une puissance seuil acceptable par l'organisme humain en phase d'endormissement et / ou de repos.

Préférentiellement, et comme l'illustrent les figures 10 à 13, ladite valeur de puissance maximale Pₘₐₓ est atteinte lors de la mise en marche dudit dispositif. En d'autres termes, dès que l'utilisateur met en fonction ledit dispositif, préférentiellement à l'aide dudit bouton de mise en marche, la source lumineuse 3 est mise à contribution et produit sa puissance maximale Pₘₐₓ. De manière alternative, et sans sortir du cadre de l'invention, il est également parfaitement envisageable que ladite valeur de puissance maximale Pₘₐₓ ne soit pas atteinte immédiatement après la mise en fonction dudit dispositif, mais plutôt après quelques secondes, par exemple afin de préserver ladite source lumineuse 3 et / ou sa source d'énergie (et donc augmenter leurs durées de vie), voire même après quelques minutes, par exemple afin de conférer un meilleur confort à l'utilisateur (notamment afin d'éviter tout éblouissement de ce dernier, ou bien pour respecter ses préférences personnelles comme cela sera détaillé par la suite).

Ainsi, ledit dispositif est avantageusement conçu pour accompagner l'utilisateur dans son endormissement, et l'aider à trouver le sommeil en diffusant dans la pièce (préférentiellement dans la chambre) une lumière rouge qui va progressivement et continûment diminuer en intensité pour finalement disparaitre, ladite disparition (ou extinction de ladite source lumineuse 3) intervenant avantageusement après l'endormissement de l'utilisateur, grâce à ladite loi prédéterminée L. En d'autres termes, ledit utilisateur va être accompagné par la lumière rouge dont l'intensité est décroissante lors de sa phase d'endormissement, ladite lumière rouge ayant pratiquement disparue avantageusement quelques minutes après l'endormissement de l'utilisateur. Ainsi, grâce audit dispositif et à sa loi prédéfinie, l'utilisateur se trouve dans des conditions optimales pour s'endormir :
- dans un premier temps, préalablement à la mise en fonction dudit dispositif, la pièce (chambre à coucher par exemple) dans laquelle se trouve l'utilisateur est éclairée au moyen de la lumière normale de la chambre, pour permettre à l'utilisateur de vaquer à ses dernières occupations avant son coucher ;
- puis le dispositif de l'invention est mis en marche et les lumières normales sont éteintes, de sorte que la pièce est alors éclairée seulement par le dispositif d'une lumière rouge apaisante ;
- l'intensité lumineuse diminue alors de manière automatique, continue et progressive, si bien que la luminosité ambiante de la pièce diminue également, de manière à placer progressivement l'utilisateur dans une ambiance lumineuse particulièrement relaxante et rassurante (notamment pour les personnes ayant peur du noir), et assurer une transition entre l'éclairage intensif tel que celui subi par l'utilisateur dans la journée et le noir total tel qu'il pourra le connaître une fois endormi;
- enfin, après par exemple un laps de temps d'une vingtaine ou une trentaine de minutes correspondant généralement au temps d'endormissement, le dispositif atteint ladite puissance sensiblement nulle et la pièce n'est alors plus éclairée par ledit dispositif.

Ainsi, tout au long de cette diminution progressive, l'utilisateur est exposé aux bienfaits de la lumière rouge, et ce même si la pièce n'est pas dans l'obscurité, comme c'est par exemple le cas dans les régions polaires ou les zones fortement urbanisées.

De manière préférentielle, ladite loi prédéfinie L est conçue pour que ladite puissance lumineuse P décroisse sensiblement continûment en fonction du temps t entre ladite valeur de puissance maximale Pₘₐₓ et ladite valeur de puissance minimale, et avantageusement entre ladite valeur de puissance maximale Pₘₐₓ et ladite valeur de puissance sensiblement nulle, éventuellement après un palier à puissance constante où la puissance lumineuse P est constante (de préférence égale à ladite valeur de puissance minimale) pendant une durée de palier prédéterminée.

En d'autres termes, la puissance P est une fonction du temps et ladite loi prédéfinie L comprend préférentiellement une fonction continue sur l'intervalle supérieur ou égal à zéro de ladite puissance lumineuse P en fonction du temps t. Ainsi, ladite loi prédéfinie L suit préférentiellement une fonction mathématique qui est continue pour n'importe quelle valeur de temps, étant entendu que le temps t court à partir du moment où ledit dispositif est mis en marche (en fonctionnement). Avantageusement, ladite fonction est continûment décroissante entre ladite valeur de puissance maximale Pₘₐₓ qui constitue son maximum et ladite puissance sensiblement nulle, qui constitue préférentiellement son minimum. En d'autres termes, ladite fonction est de préférence une fonction monotone au sens large (s'il y a un ou plusieurs paliers) ou monotone stricte (s'il n'y a aucun palier), et décroissante, ce qui signifie qu'elle ne fait que décroître au cours du temps (avec ou sans palier), et qu'elle n'est de manière préférentielle jamais croissante (notamment après que ladite puissance lumineuse ait atteint Pₘₐₓ). En d'autres termes encore, la puissance P émise par ladite source lumineuse 3 diminue sans cesse de manière progressive, éventuellement après un palier à puissance constante pendant lequel la puissance ne varie sensiblement pas, de sorte que dans ce cas l'utilisateur bénéficie d'une lumière rouge de puissance fixe pendant une durée donnée ou choisie, puis l'intensité diminue en fonction de la rapidité d'extinction définie ou choisie, sans que cette baisse d'intensité régulière ne soit de préférence affectée par un palier. Ainsi, avantageusement, pendant toute la durée d'utilisation du dispositif d'aide à l'endormissement (c'est-à-dire quel que soit le temps t), ladite puissance P ne fait que décroître (ou rester stable s'il y a un ou plusieurs paliers), de manière automatique et progressive en fonction du temps t, de sorte que la puissance lumineuse émise par ladite source lumineuse 3 à un instant T est toujours supérieure (ou égale, s'il y a un ou plusieurs paliers) à la puissance lumineuse émise à un instant ultérieur T+1. En d'autres termes, plus le temps t s'écoule, plus la puissance lumineuse émise par ledit dispositif diminue, de préférence sans jamais augmenter (notamment après avoir atteint Pₘₐₓ). Alternativement, il est toutefois envisageable que la loi prédéfinie ne prévoit aucun palier à puissance constante. Ainsi, grâce à ladite loi prédéfinie L, ladite puissance P est avantageusement globalement (par exemple s'il y a un ou plusieurs paliers) et/ou strictement (par exemple s'il n'y a aucun palier) décroissante au cours du temps t, et jamais croissante, sauf par exemple à la mise en marche du dispositif, ou pendant un certain délai après la mise en marche du dispositif, et notamment jamais croissante après avoir atteint ladite valeur de puissance maximale Pₘₐₓ.

De manière avantageuse, et comme l'illustrent les figures 10 et 11, ladite fonction est une fonction affine, c'est-à-dire que ladite loi prédéfinie L évolue selon une droite. Le coefficient directeur de ladite droite, c'est-à-dire la pente de la droite, est alors négatif dans la mesure où ladite fonction est décroissante et constitue d'ailleurs un paramètre important de ladite loi prédéfinie L. En outre, d'un point de vu mathématique, ladite valeur de puissance maximale Pₘₐₓ représente l'ordonnée à l'origine de ladite fonction affine, c'est-à-dire le point de départ de la dite droite, comme on peut le voir sur les figures 10 et 11.

Préférentiellement, ladite loi prédéfinie L est conçue pour que ladite source lumineuse 3 atteigne ladite valeur de puissance minimale, et avantageusement ladite valeur de puissance sensiblement nulle, à partir de ladite valeur de puissance maximale Pₘₐₓ après qu'une durée prédéterminée d'extinction dₑ se soit écoulée. En d'autres termes, ledit dispositif est conçu pour automatiquement atteindre sa puissance minimale et préférentiellement une puissance sensiblement nulle après une certaine durée prédéfinie d'extinction dₑ, c'est-à-dire après un certain laps de temps défini à l'avance.

De manière avantageuse, ladite durée prédéterminée d'extinction dₑ est comprise entre 15 et 40 minutes, par exemple entre 20 et 30 minutes, et est avantageusement sensiblement égaie à 30 minutes, ce qui correspond avantageusement à la durée d'endormissement moyen d'un utilisateur humain.

Préférentiellement, ledit dispositif comprend une minuterie conçue pour décompter automatiquement ladite durée prédéterminée d'extinction dₑ à partir de la mise en marche dudit dispositif t₀. En d'autres termes, ledit dispositif est capable, de manière autonome, de calculer automatiquement le temps qui s'écoule à partir du moment où ledit dispositif est mis en fonction, préférentiellement par l'utilisateur, par exemple à l'aide d'un chronomètre, d'une horloge, d'un compteur, etc. ou de tout autre moyen apte à déterminer automatiquement et de manière autonome le moment à l'issu duquel ladite puissance P doit être sensiblement nulle. Naturellement, sans sortir du cadre de l'invention, il est parfaitement envisageable que ladite durée prédéterminée d'extinction dₑ soit calculée non pas à partir de la mise en fonction dudit dispositif mais plutôt à partir d'un autre moment, comme par exemple un moment qui serait décidé par l'utilisateur lui-même et qui pourrait correspondre à un appui sur un bouton et par exemple à un second appui bref sur ledit bouton de mise en marche.

De manière avantageuse, ledit dispositif comprend un moyen d'extinction conçu pour mettre automatiquement ledit dispositif à l'arrêt à l'issue de ladite durée prédéterminée d'extinction d_{e.} En d'autres termes, ledit dispositif est avantageusement conçu pour être automatiquement mis à l'arrêt, c'est-à-dire mis hors service et / ou hors tension, à l'issu de ladite durée prédéterminée d'extinction dₑ, c'est-à-dire préférentiellement lorsque ledit dispositif atteint ladite puissance sensiblement nulle. II est ainsi remarquablement possible de faire des économies d'énergie, dans la mesure où aucun composant ne reste en veille.

Préférentiellement, ledit dispositif comprend un moyen de modification et / ou de création de loi permettant à l'utilisateur de modifier ladite loi prédéfinie L et / ou de créer une nouvelle loi. Ainsi, ledit dispositif est préférentiellement conçu pour que ladite loi prédéfinie L puisse être modifiée par l'utilisateur s'il le souhaite, notamment afin que ce dernier puisse l'adapter à ses habitudes, et en particulier à ses habitudes de sommeil, d'endormissement et / ou de repos. Alternativement ou complémentairement, ledit dispositif est également avantageusement conçu pour permettre à l'utilisateur de créer une nouvelle loi destinée à remplacer ladite loi prédéfinie L. A cette fin, ledit dispositif comprend un ou plusieurs éléments, outils, dispositifs, ou moyens capables d'interagir d'une part avec l'utilisateur et d'autre part avec ledit dispositif et plus particulièrement avec ladite unité de commande, afin de modifier et / ou remplacer ladite loi prédéfinie L. Ainsi, ledit dispositif comprend par exemple une deuxième interface utilisateur qui peut comprendre de manière illustrative et non limitative un ou plusieurs boutons, une molette, un clavier, un logiciel, une application, etc.

De manière avantageuse, ledit moyen de modification et / ou de création de loi est un moyen de modification de ladite durée prédéterminée d'extinction dₑ, c'est-à-dire que ledit moyen de modification et / ou de création de loi permet à l'utilisateur de modifier ladite durée prédéterminée d'extinction dₑ. En d'autres termes, il est possible pour l'utilisateur de personnaliser ledit dispositif, et plus particulièrement le fonctionnement de ce dernier, en intervenant directement sur ladite loi prédéfinie L, par exemple en modifiant ladite durée prédéterminée d'extinction dₑ, c'est-à-dire en modifiant le délai d'extinction dudit dispositif et / ou le délai après lequel ledit dispositif atteint ladite puissance sensiblement nulle. De manière illustrative et non limitative, la figure 11 illustre une modification de ladite durée prédéterminée d'extinction dₑ par rapport à la figure 10, et plus précisément un allongement de cette durée, par exemple afin de la faire passer de 22 minutes à 30 minutes, dans le cas où l'utilisateur considérerait que la durée initiale de 22 minutes est trop courte et ne lui permet pas de s'endormir avant l'extinction dudit dispositif. Ainsi, il est avantageusement possible à l'utilisateur d'adapter ladite durée prédéterminée d'extinction dₑ à ses habitudes et en particulier à son temps d'endormissement, notamment de manière à ce qu'il puisse s'endormir avant que ledit dispositif ne se soit automatiquement mis hors service et / ou avant que ladite source lumineuse 3 n'ait atteinte ladite puissance sensiblement nulle. L'utilisateur peut alors d'une manière tout à fait remarquable bénéficier des bienfaits de la lumière rouge émise par ledit dispositif jusqu'à son endormissement complet, et ce quelle que soit la durée habituelle de celui-ci.

De manière alternative ou complémentaire, ledit moyen de modification et / ou de création de loi est un moyen de modification de la valeur de puissance maximale Pₘₐₓ, c'est-à-dire que ledit moyen de modification et / ou de création de loi permet à l'utilisateur de modifier la valeur de ladite puissance maximale Pₘₐₓ. En d'autres termes, il est possible pour l'utilisateur de personnaliser ledit dispositif, et plus particulièrement le fonctionnement de ce dernier, en intervenant directement sur ladite loi prédéfinie L, par exemple en modifiant ladite valeur de puissance maximale Pₘₐₓ, c'est-à-dire en modifiant la quantité de lumière qui est émise par ledit dispositif et plus précisément par ladite source lumineuse 3. De manière illustrative et non limitative, la figure 11 illustre une modification de ladite valeur de puissance maximale Pₘₐₓ par rapport à la figure 10, et plus précisément une diminution de cette dernière, par exemple afin de la faire passer de 885 lux à 500 lux, dans le cas où l'utilisateur considérerait que la puissance maximale initiale de 885 lux est trop importante et ne lui permet pas de s'endormir et / ou de se relaxer. D'un point de vue mathématique, dans le cas où ladite loi prédéfinie suit une fonction affine, la modification de ladite valeur de puissance maximale Pₘₐₓ revient à modifier l'ordonnée à l'origine de la droite. Ainsi, il est avantageusement possible à l'utilisateur d'adapter ladite valeur de puissance maximale Pₘₐₓ à ses habitudes, ses sensibilités sensorielles ou encore ses préférences.

Dans le cas particulier où ladite loi prédéfinie L suit une décroissance définie par une fonction affine, il est ainsi avantageusement possible à l'utilisateur, en modifiant ladite valeur de puissance maximale Pₘₐₓ et / ou en modifiant ladite durée prédéterminée d'extinction dₑ, de modifier la pente de la droite, c'est-à-dire de modifier son coefficient directeur, comme on peut le voir sur les figures 10 et 11 par exemple.

De manière alternative ou complémentaire, il est également parfaitement envisageable, et ce sans sortir du cadre de l'invention, que ledit moyen de modification et / ou de création de loi soit un moyen de modification de l'instant où ladite valeur de puissance maximale Pₘₐₓ est atteinte, c'est-à-dire que ledit moyen de modification et / ou de création de loi permette à l'utilisateur de modifier l'instant où ladite valeur de puissance maximale Pₘₐₓ est atteinte, par exemple afin de choisir un autre moment que le moment d'allumage dudit dispositif, ou encore la forme de décroissance que va suivre ladite loi prédéfinie L, comme l'illustrent par exemple les figures 12 et 13. Plus précisément, il est parfaitement envisageable pour l'utilisateur de modifier la fonction affine de la figure 10 par exemple prédéfinie comme réglage de base (ou réglage d'usine) dudit dispositif pour la transformer en une courbe décroissante de forme convexe telle que celle visible à la figure 12, ou bien encore en une courbe de forme concave telle que celle visible à la figure 13 si une telle décroissance est d'avantage adaptée à ses habitudes et / ou ses goûts personnels.

Selon le mode de réalisation préférentiel visible à la figure 9, ledit dispositif comprend avantageusement un module de communication à distance comme par exemple une antenne, une prise Ethernet, une prise USB, etc. Avantageusement, le module de communication à distance est conçu pour communiquer avec un terminal de commande à distance 4 tel qu'une une télécommande, un ordinateur, ou avantageusement une tablette ou un ordiphone tel qu'illustré à la figure 9. En d'autres termes, ledit dispositif est alors en communication avec ledit terminal de commande à distance 4, que ce soit de manière continue ou ponctuelle, de manière filaire ou non, afin de pouvoir recevoir des informations, et préférentiellement des données informatiques de la part dudit terminal de commande à distance 4. Préférentiellement, ledit terminal de commande à distance 4 embarque ledit moyen de modification et / ou de création de loi., par sous la forme d'une application ou d'un logiciel.

De manière avantageuse, ledit terminal de commande à distance 4 comprend ou forme par lui-même ladite deuxième interface utilisateur qui comprend alors préférentiellement un écran et avantageusement d'un écran tactile, permettant alors à l'utilisateur dudit dispositif d'interagir facilement et intuitivement avec ledit dispositif par l'intermédiaire dudit terminal de commande à distance 4, par exemple à l'aide de menus, d'icônes, de fenêtres, ou encore à partir de graphiques comme on peut le voir par exemple sur la figure 9. Préférentiellement, et de manière connue en tant que telle, ladite deuxième interface utilisateur est contrôlée au moyen d'une application exécutée par ledit terminal de commande à distance.

Préférentiellement, ladite communication est une communication sans fil comme par exemple une communication de type Bluetooth^{®}, Wifi^{®}, etc. bien connue en tant que telle et aisément disponible sur des terminaux de commande à distance portatifs de type tablette ou ordiphone. Dans ce cas, ledit dispositif comprend alors bien entendu un module correspondant apte à recevoir et éventuellement émettre des signaux compatibles avec le protocole de communication utilisé, comme par exemple une antenne.

Selon le mode de réalisation préférentiel visible sur les figures, ledit dispositif comprend un boîtier 1 à l'intérieur duquel est disposée ladite source lumineuse 3. En d'autres termes, ledit dispositif comprend un carter ou une enveloppe destiné à accueillir ladite source lumineuse 3. Avantageusement, ledit boîtier est conçu pour l'une et / ou l'autre des fonctionnalités suivantes :
- pouvoir être tenu dans ou à la main,
- protéger ladite source lumineuse 3, par exemple de l'environnement (poussière, humidité, chute d'objets, etc.),
- maintenir et orienter cette dernière de manière à ce que les rayons lumineux produits par ladite source lumineuse 3 soient sensiblement orientés verticalement et de préférence vers le plafond de la pièce dans laquelle se trouve ledit dispositif,
- optimiser la diffusion de la lumière émise par ladite source lumineuse 3, par exemple en comprenant un diffuseur 2 comme cela sera détaillé par la suite,
- alimenter en énergie ladite source lumineuse, par exemple en contenant également une source d'alimentation comme cela sera détaillé par la suite.

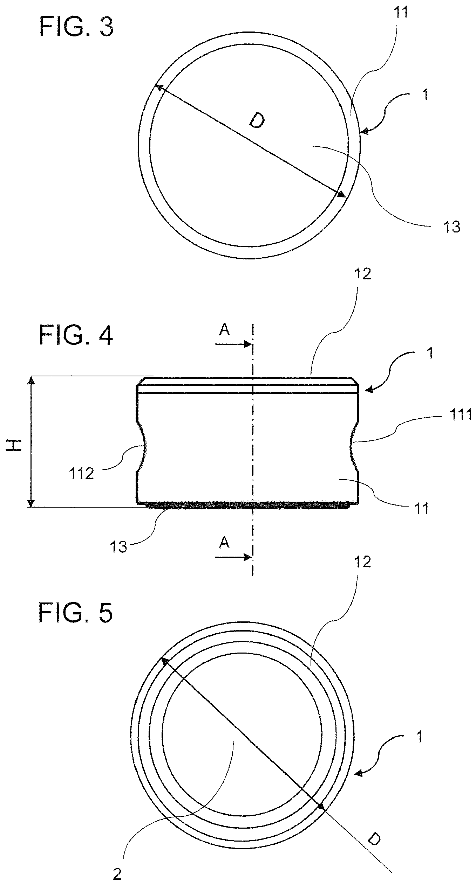

Préférentiellement, ledit boîtier 1 est de forme sensiblement cylindrique et son diamètre extérieur D est préférentiellement compris entre 50 mm et 80 mm et est avantageusement sensiblement égal à 70 mm, comme cela est illustré sur la figure 3 ou 5 notamment, tandis que sa hauteur H est préférentiellement comprise entre 20 mm et 40 mm et est avantageusement sensiblement égale à 35 mm, comme l'illustre par exemple la figure 4. Ainsi, ledit dispositif est remarquablement ergonomique et compact, de sorte qu'il est aisé pour l'utilisateur d'une part de le placer n'importe où dans sa chambre à coucher, comme par exemple sur sa table de nuit, et d'autre part de l'emmener avec lui, par exemple lors de voyages où l'utilisateur ne dort pas dans sa chambre habituelle, comme par exemple dans une chambre d'hôtel. Ainsi, grâce à la remarquable compacité dudit dispositif, l'utilisateur pourra retrouver tous les bienfaits de la lumière rouge émise par ledit dispositif, et ce y compris dans une chambre d'hôtel, dans la mesure où ledit dispositif est particulièrement adapté à être transporté dans un sac de voyage par exemple. Cette transportabilité optimisée dudit dispositif est alors d'autant plus appréciable pour l'utilisateur que c'est généralement lors des voyages, lorsque l'utilisateur de dort pas dans sa chambre habituelle, qu'il peut le plus souvent avoir du mal à trouver le sommeil et / ou se relaxer. En outre, les dimensions dudit dispositif sont également remarquablement adaptées à l'ergonomie humaine, et en particulier à la main de l'homme, de sorte qu'il est possible, et même aisé pour l'utilisateur de saisir ledit dispositif à une seule main, mais également de le contrôler, notamment par l'intermédiaire de ladite première interface utilisateur d'une seule main.

Toujours selon le mode de réalisation préférentiel visible sur les différentes figures, ledit dispositif comprend un diffuseur 2 conçu pour optimiser la diffusion de la lumière émise par ladite source lumineuse 3, c'est-à-dire un moyen permettant d'améliorer la diffusion de ladite lumière.

Selon le mode de réalisation préférentiel précédemment décrit, ledit diffuseur 2 comprend avantageusement un disque translucide, et / ou éventuellement un disque transparent. En d'autres termes, ledit diffuseur 2 forme un élément optique conçu pour améliorer la diffusion de la lumière issue de ladite source lumineuse 3. Ainsi, grâce audit diffuseur 2, il est par exemple possible, d'améliorer l'homogénéité de la lumière diffusée dans la pièce où se situe ledit dispositif. Finalement, ledit diffuseur 2 est avantageusement conçu pour transformer les rayons lumineux de ladite source lumineuse 3 en une lumière diffuse et homogène.

Outre cette fonction de diffusion, ledit diffuseur est également avantageusement conçu pour protéger ladite source lumineuse, notamment de l'environnement et des agressions de ce dernier comme par exemple la poussière, les projections d'eau, etc. en formant avantageusement une paroi de protection, éventuellement étanche, située au-dessus de ladite source de lumière 3. Préférentiellement ledit diffuseur 2 forme une partie de la portion plane supérieure du cylindre que forme ledit boîtier 1.

Préférentiellement, le diamètre D₂ dudit diffuseur 2 est compris entre 50 mm et 70 mm et est avantageusement sensiblement égal à 62,50 mm, notamment de manière à pouvoir être inséré à l'intérieur du boîtier 1, tandis que son épaisseur est comprise entre 1 et 3 mm et est avantageusement sensiblement égale à 1,5 mm, cette dernière étant particulièrement adaptée pour diffuser la lumière émise par ladite source lumineuse 3 de manière optimale.

Plus précisément, ledit diffuseur 2 comprend une face intérieure 21 faisant face à ladite source lumineuse 3 et une face extérieure 22 opposée, et donc avantageusement en contact avec l'environnement. En d'autres termes, ledit diffuseur 2 reçoit la lumière de ladite source lumineuse 3 par sa face intérieure 21 et la diffuse à l'environnement par sa face extérieure 22, comme on peut le voir sur la figure 6 par exemple. Préférentiellement, et compte tenu de l'implantation dudit diffuseur 2 dans le boîtier 1, ladite face intérieure 21 est la face inférieure et ladite face extérieure 22 est la face supérieure. De manière avantageuse, ladite face intérieure 21 présente un état de surface plus lisse que l'état de surface de ladite surface extérieure 22, ce qui permet, de manière remarquable d'optimiser la diffusion de ladite lumière. En d'autres termes, lesdites faces intérieure et extérieure 21, 22 dudit diffuseur n'ont pas le même état de surface de manière à optimiser et améliorer la diffusion de la lumière émise par ladite source lumineuse. Plus précisément, ladite face intérieure 21 recevant la lumière présente un état de surface qui est relativement lisse tandis que ladite face extérieure 22 diffusant la lumière présente un état de surface qui est relativement granuleux.

De manière avantageuse, ledit diffuseur 2 est réalisé en un matériau comprenant au moins du polycarbonate et un produit diffusant, notamment afin de modifier les propriétés naturelles de transparence du polycarbonate. Alternativement, ou complémentairement, d'autres matériaux pourront être utilisés pour réaliser ledit diffuseur 2, et ce sans sortir du cadre de l'invention, comme par exemple du poly(méthacrylate de méthyle) (PMMA), du polyester (PES), ou du polyépoxyde, ces matériaux étant préférentiellement combinés à un produit diffusant.

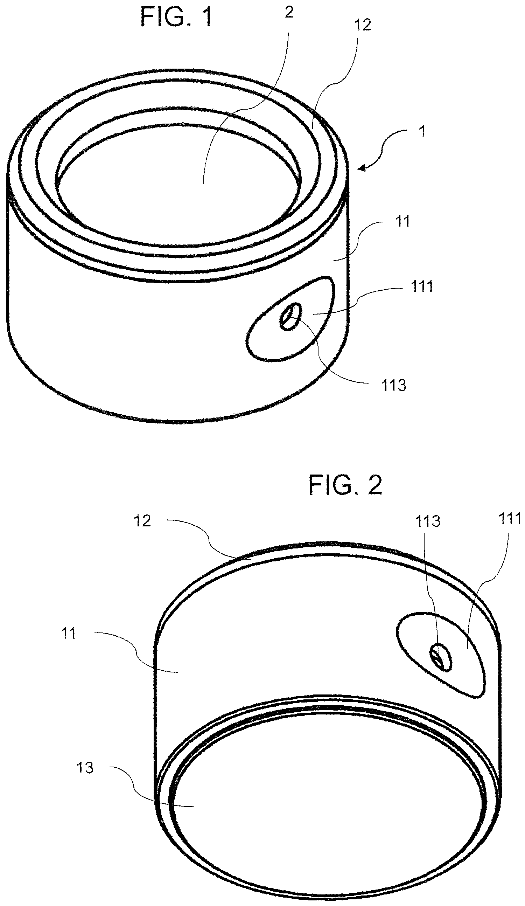

Préférentiellement, ledit boîtier 1 comprend un corps principal 11 formé d'une portion de tube cylindrique, comme on peut le voir sur les figures 1 à 6, ledit corps principal 11 étant conçu pour accueillir ledit diffuseur 2.

Selon le mode de réalisation préférentiel visible notamment sur la figure 6, ledit boîtier 1, et plus précisément ledit corps principal 11, comprend un compartiment de diffusion 15 comprenant lui-même ledit diffuseur 2. Préférentiellement, ledit compartiment de diffusion 15 se présente sous la forme d'une cavité, avantageusement cylindrique située entre ledit diffuseur 2 et ladite source lumineuse 3 à l'intérieure de laquelle ladite lumière émise par ladite source lumineuse 3 circule.

Préférentiellement, ledit boîtier 1 comprend un couvercle 12 conçu pour interagir d'une part avec ledit corps principal 11 et d'autre part avec ledit diffuseur 2. Plus précisément, comme on peut le voir sur la figure 6, ledit couvercle 12 est conçu pour maintenir en place ledit diffuseur 2 au sein dudit corps principal 11 tout en laissant passer la lumière issue dudit diffuseur 2. A cette fin, ledit couvercle 12 se présente préférentiellement sous la forme d'une bague indémontable, sertie avec un joint torique à l'extrémité supérieure dudit corps principal 11 au-dessus dudit diffuseur 2, comme on peut le voir sur la figure 6. Plus précisément, ledit couvercle 12 est alors avantageusement équipé d'un filetage conçu pour correspondre avec un taraudage situé à l'intérieur dudit corps principal 11. De manière avantageuse, comme on peut le voir sur la figure 6, ledit diffuseur 2 est alors en appui sur un épaulement dudit corps principal 11 de sorte que le diffuseur 2 est totalement immobilisé. De préférence, ledit couvercle 12 est indémontable, étant entendu qu'il est alternativement envisageable qu'il soit démontable, par exemple à l'aide d'une opération de dévissage, afin de permettre d'accéder audit diffuseur 2, par exemple pour une opération de nettoyage ou de remplacement de ce dernier.

Toujours selon ce même mode de réalisation, ledit boîtier 1 comprend également un compartiment d'alimentation 14 conçu pour alimenter en énergie ladite source lumineuse 3, ce dernier comportant avantageusement une source d'alimentation électrique, telle qu'une batterie ou une pile par exemple. En d'autres termes, ledit compartiment d'alimentation 14 est alors avantageusement conçu pour accueillir une source d'alimentation de ladite source lumineuse 3, et préférentiellement une source d'alimentation électrique. Avantageusement, ladite source d'alimentation est une source rechargeable comme par exemple une batterie.

De manière avantageuse, et comme on peut le voir sur les figures 1 et 2 notamment, ledit boîtier 1, et plus précisément ledit compartiment d'alimentation 14, comprend une première ouverture 113 orientée transversalement à la paroi du cylindre ou du tube dudit corps principal 11. Préférentiellement, ladite première ouverture 113 est conçue pour accueillir ladite première interface utilisateur permettant à l'utilisateur d'interagir avec ledit dispositif et plus précisément ledit bouton de mise en marche. De manière avantageuse, ladite première ouverture 113 comprend un trou circulaire réalisé perpendiculairement à la paroi cylindrique dudit boîtier 1.

Préférentiellement, ladite première ouverture 113 se situe au centre d'un premier lamage 111 tel qu'on peut le voir sur les figures 1, 2 et 4, ledit lamage 111 étant avantageusement conçu pour d'une part améliorer l'ergonomie du dispositif vis-à-vis des doigts de l'utilisateur et d'autre part limiter le risque d'appui intempestif sur ledit bouton de mise en marche, notamment lorsque ledit dispositif se trouve dans un sac de transport.

De manière avantageuse, et comme on peut le voir sur la figure 6, ledit boîtier 1, et plus précisément ledit compartiment d'alimentation 14, comprend une deuxième ouverture 114 située transversalement à la paroi du cylindre ou du tube dudit corps principal 11. Préférentiellement, ladite deuxième ouverture 114 est conçue pour accueillir un moyen de recharge de ladite source d'alimentation électrique, permettant à l'utilisateur de recharger ledit dispositif et plus précisément ladite source d'alimentation électrique. A titre d'exemple illustratif et non limitatif, ledit moyen de recharge peut comprendre une prise de type micro-USB connectée à un chargeur que l'utilisateur peut connecter sur une prise électrique domestique. De manière avantageuse, ladite deuxième ouverture 114 comprend un trou circulaire réalisé perpendiculairement à la paroi cylindrique dudit boîtier 1.

Préférentiellement, ladite deuxième ouverture 114 se situe au centre d'un deuxième lamage 112 tel qu'on peut le voir sur la figure 4, ledit lamage 112 étant avantageusement conçu pour d'une part améliorer l'ergonomie du dispositif vis-à-vis des doigts de l'utilisateur et d'autre part limiter le risque de détérioration de la connectique destinée à recevoir ledit moyen de recharge, notamment lorsque ledit dispositif se trouve dans un sac de transport.

De manière avantageuse, lesdites première et deuxième ouvertures 113, 114 ainsi que lesdits premier et deuxième lamages 111, 112 associés sont diamétralement opposés et sont par conséquent symétriques par rapport à l'axe de révolution dudit boîtier 1.

Préférentiellement, ledit boîtier 1 comprend un bouchon 13 conçu pour fermer, avantageusement d'une manière relativement étanche, ledit compartiment d'alimentation 14, tel que l'on peut le voir sur la figure 2 ou 6. Ledit bouchon 13 est avantageusement indémontable.

Avantageusement, ledit bouchon 13 est plein et constitue la base du cylindre formé par ledit boîtier 1.

Préférentiellement, ledit boîtier 1 est pourvu d'une embase qui lui permet de reposer de façon stable sur une surface plane, telle que la surface d'une table de nuit, avec la source de lumière orientée vers le dessus, préférentiellement selon une incidence verticale avantageusement orientée du bas vers le haut.

De manière avantageuse, et comme on peut le voir sur les figures 2 et 6, ladite embase est formée par ledit bouchon 13 qui est alors également conçu pour servir de socle ou de base audit dispositif, c'est-à-dire qu'il est destiné à supporter au moins le poids dudit dispositif. En effet, comme on peut le voir sur la figure 2 notamment, ledit bouchon 13 servira avantageusement de surface d'appui pour poser ledit dispositif sur la table de chevet de l'utilisateur par exemple. A cette fin, ledit bouchon est préférentiellement fabriqué dans un matériau particulièrement robuste comme par exemple un matériau plastique.

De manière avantageuse, et comme on peut le voir sur la figure 6 notamment, lesdits compartiments d'alimentation 14 et de diffusion 15 sont coaxiaux avec ladite source lumineuse 3 disposée entre eux, ce qui permet d'une manière remarquable d'améliorer la compacité dudit dispositif, notamment en disposant ladite source lumineuse entre sa source d'alimentation et le diffuseur 2. Entre d'autres termes, ladite source lumineuse 3 est disposée de manière à séparer lesdits compartiments d'alimentation 14 et de diffusion 15.

L'invention concerne également en tant que tel un système d'aide à l'endormissement d'un utilisateur comprenant un dispositif conforme à celui précédemment décrit ainsi qu'un terminal de commande à distance 4 qui embarque ledit moyen de modification et /ou de création de loi, ledit terminal de commande à distance 4 étant conçu pour communiquer avec un module de communication à distance dudit dispositif.

La description concerne également en tant que tel un procédé d'endormissement d'un utilisateur dans lequel on soumet un utilisateur à une lumière monochromatique rouge favorisant l'endormissement de l'utilisateur à partir d'une source lumineuse 3 caractérisé en ce que l'on fait automatiquement et progressivement décroître au cours du temps t la puissance lumineuse P émise par ladite source lumineuse 3 de manière à ce que ladite puissance P évolue automatiquement entre une valeur de puissance lumineuse maximale Pₘₐₓ et une valeur de puissance minimale qui est de préférence sensiblement nulle, la puissance lumineuse émise par ladite source lumineuse 3 à un instant T étant toujours supérieure ou égale à la puissance lumineuse émise à un instant ultérieur T+1.

Préférentiellement, ledit procédé comprend en outre une étape de modification de ladite loi prédéfinie L et / ou de création d'une nouvelle loi destinée à remplacer ladite loi prédéfinie L.

### POSSIBILITE D'APPLICATION INDUSTRIELLE

L'invention trouve son application industrielle dans la fabrication et l'utilisation de dispositifs d'aide à l'endormissement au moyen de lumière.

## Revendications

1. - Dispositif d'aide à l'endormissement d'un utilisateur, comprenant une source lumineuse (3) conçue pour émettre une lumière sensiblement monochromatique rouge favorisant l'endormissement de l'utilisateur, ledit dispositif comprenant une unité de commande conçue pour contrôler automatiquement ladite source lumineuse (3) conformément à une loi prédéfinie (L), ledit dispositif étant **caractérisé en ce que** selon ladite loi prédéfinie (L), la puissance lumineuse (P) émise par ladite source lumineuse (3) est progressivement décroissante au cours du temps (t) de manière à évoluer automatiquement entre une valeur de puissance maximale (Pmax) et une valeur de puissance minimale, la puissance lumineuse émise par ladite source lumineuse (3) à un instant (T) étant toujours supérieure ou égale à la puissance lumineuse émise à un instant ultérieur (T+1) pendant toute la durée d'utilisation dudit dispositif d'aide à l'endormissement, ladite puissance lumineuse (P) n'étant jamais croissante après avoir atteint ladite valeur de puissance maximale (Pmax).

2. - Dispositif d'aide à l'endormissement selon la revendication précédente **caractérisé en ce que** ladite valeur de puissance minimale est une valeur sensiblement nulle.

3. - Dispositif d'aide à l'endormissement selon l'une des revendications précédentes **caractérisé en ce que** ladite loi prédéfinie (L) est conçue pour que ladite puissance lumineuse (P) décroisse sensiblement continûment en fonction du temps (t) entre ladite valeur de puissance maximale (Pmax) et ladite valeur de puissance minimale.

4. - Dispositif d'aide à l'endormissement selon l'une des revendications précédentes **caractérisé en ce que** ladite valeur de puissance lumineuse maximale (Pmax) est supérieure à 600 lux et est avantageusement sensiblement égale à 885 lux, ladite source de lumière comprenant de préférence au moins une diode électroluminescente (LED).

5. - Dispositif d'aide à l'endormissement selon l'une des revendications précédentes **caractérisé en ce que** ladite valeur de puissance maximale (Pmax) est atteinte lors de la mise en marche dudit dispositif.

6. - Dispositif d'aide à l'endormissement selon l'une des revendications précédentes **caractérisé en ce que** ladite loi prédéfinie (L) est conçue pour que ladite source lumineuse (3) atteigne ladite valeur de puissance minimale à partir de ladite valeur de puissance maximale (Pₘₐₓ) après qu'une durée prédéterminée d'extinction (dₑ) se soit écoulée, ladite durée prédéterminée d'extinction (dₑ) étant de préférence comprise entre 15 et 40 minutes et est plus préférentiellement encore sensiblement égale à 30 minutes, le dispositif d'aide à l'endormissement comprenant également préférentiellement une minuterie conçue pour décompter automatiquement ladite durée prédéterminée d'extinction (dₑ) à partir de la mise en marche dudit dispositif (t₀), le dispositif d'aide à l'endormissement comprenant avantageusement en outre un moyen d'extinction conçu pour mettre automatiquement ledit dispositif à l'arrêt à l'issue de ladite durée prédéterminée d'extinction (dₑ).

7. - Dispositif d'aide à l'endormissement selon l'une des revendications précédentes **caractérisé en ce qu'**il comprend un moyen de modification et / ou de création de loi permettant à l'utilisateur de modifier ladite loi prédéfinie (L) et / ou de créer une nouvelle loi destinée à remplacer ladite loi prédéfinie (L), le dispositif d'aide à l'endormissement comprenant de préférence également un module de communication à distance conçu pour communiquer avec un terminal de commande à distance (4) qui embarque ledit moyen de modification et / ou de création de loi, ladite communication étant préférentiellement une communication sans fil.

8. - Dispositif d'aide à l'endormissement selon la revendication précédente et la revendication 6 **caractérisé en ce que** ledit moyen de modification et / ou de création de loi permet à l'utilisateur de modifier ladite durée prédéterminée d'extinction (de).

9. - Dispositif d'aide à l'endormissement selon l'une des revendications 7 ou 8 et selon la revendication 4 et / ou 5 **caractérisé en ce que** ledit moyen de modification et / ou de création de loi permet à l'utilisateur de modifier la valeur de puissance maximale (Pmax) et / ou l'instant où ladite valeur de puissance maximale (Pmax) est atteinte.

10. - Dispositif d'aide à l'endormissement selon l'une des revendications précédentes **caractérisé en ce qu'**il comprend un boîtier (1) à l'intérieur duquel est disposée ladite source lumineuse (3), ledit boîtier (1) étant de forme sensiblement cylindrique.

11. -Dispositif d'aide à l'endormissement selon l'une des revendications précédentes **caractérisé en ce qu'**il comprend un diffuseur (2) conçu pour optimiser la diffusion de la lumière émise par ladite source lumineuse (3), ledit diffuseur (2) comprenant de préférence un disque translucide, ledit diffuseur (2) comprenant préférentiellement une face intérieure (21) faisant face à ladite source lumineuse (3) et une face extérieure (22) opposée, ladite face intérieure (21) présentant un état de surface plus lisse que l'état de surface de ladite surface extérieure (22), ledit diffuseur (2) étant avantageusement réalisé en un matériau comprenant au moins du polycarbonate et un produit diffusant.

12. - Dispositif d'aide à l'endormissement selon les revendications 10 et 11 **caractérisé en ce que** ledit boîtier (1) comprend un compartiment de diffusion (15) comprenant lui-même ledit diffuseur (2), ainsi qu'un compartiment d'alimentation (14) conçu pour alimenter en énergie ladite source lumineuse (3), lesdits compartiments d'alimentation (14) et de diffusion (15) étant coaxiaux avec ladite source lumineuse (3) disposée entre eux.

13. - Système d'aide à l'endormissement d'un utilisateur comprenant un dispositif conforme à l'une quelconque des revendications 7 à 9 ainsi qu'un terminal de commande à distance (4) qui embarque ledit moyen de modification et / ou de création de loi, ledit terminal de commande à distance (4) étant conçu pour communiquer avec un module de communication à distance dudit dispositif.

## Patentansprüche

1. - Einschlafhilfevorrichtung für eine Benutzer, mit einer Lichtquelle (3), die dazu ausgelegt ist, ein im Wesentlichen monochromatisches rotes Licht auszusenden, das das Einschlafen des Benutzers fördert, wobei die Vorrichtung eine Steuereinheit umfasst, die dazu ausgelegt ist, die Lichtquelle (3) gemäß einem vordefinierten Gesetz (L) automatisch zu steuern, wobei die Vorrichtung **dadurch gekennzeichnet ist, dass** gemäß dem vordefinierten Gesetz (L), die von der Lichtquelle (3) abgestrahlte Lichtleistung (P) im Laufe der Zeit (t) allmählich abnimmt, so dass sie sich automatisch zwischen einem maximalen Leistungswert (Pₘₐₓ) und einem minimalen Leistungswert entwickelt, wobei die von der Lichtquelle (3) zu einem Zeitpunkt (T) ausgestrahlte Lichtleistung während der gesamten Nutzungsdauer der Einschlafhilfevorrichtung immer größer oder gleich der zu einem späteren Zeitpunkt (T+1) ausgestrahlten Lichtleistung ist, wobei die Lichtleistung (P) nach Erreichen des maximalen Leistungswerts (Pmax) niemals ansteigt.

2. - Einschlafhilfevorrichtung nach dem vorhergehenden Anspruch, **dadurch gekennzeichnet, dass** der minimale Leistungswert ein Wert von im Wesentlichen Null ist.

3. - Einschlafhilfevorrichtung nach einem der vorhergehenden Ansprüche, **dadurch gekennzeichnet, dass** das vordefinierte Gesetz (L) so ausgelegt ist, dass die Lichtleistung (P) im Wesentlichen kontinuierlich als eine Funktion der Zeit (t) zwischen dem maximalen Leistungswert (Pₘₐₓ) und dem minimalen Leistungswert abnimmt.

4. - Einschlafhilfevorrichtung nach einem der vorhergehenden Ansprüche, **dadurch gekennzeichnet, dass** der maximale Lichtleistungswert (Pₘₐₓ) größer als 600 Lux ist und vorteilhafterweise im Wesentlichen gleich 885 Lux ist, wobei die Lichtquelle vorzugsweise mindestens eine lichtemittierende Diode (LED) umfasst.

5. - Einschlafhilfevorrichtung nach einem der vorhergehenden Ansprüche, **dadurch gekennzeichnet, dass** der maximale Leistungswert (Pₘₐₓ) beim Einschalten der Vorrichtung erreicht wird.

6. - Einschlafhilfevorrichtung nach einem der vorhergehenden Ansprüche, **dadurch gekennzeichnet, dass** das vordefinierte Gesetz (L) so ausgelegt ist, dass die Lichtquelle (3) den minimalen Leistungswert ausgehend von dem maximalen Leistungswert (Pₘₐₓ) erreicht, nachdem eine vorbestimmte Ausschaltdauer (dₑ) verstrichen ist, wobei die vorbestimmte Ausschaltdauer (dₑ) vorzugsweise zwischen 15 und 40 Minuten beträgt und noch bevorzugter im Wesentlichen 30 Minuten beträgt, die Einschlafhilfevorrichtung vorzugsweise auch einen Timer umfasst, der dazu ausgelegt ist, die vorbestimmte Ausschaltdauer (dₑ) ab dem Einschalten der Vorrichtung (t₀) automatisch herunterzuzählen, wobei die Einschlafhilfevorrichtung vorteilhafterweise weiterhin ein Ausschaltmittel umfasst, das dazu ausgelegt ist, die Vorrichtung nach Ablauf der vorbestimmten Ausschaltdauer (dₑ) automatisch abzuschalten.

7. - Einschlafhilfevorrichtung nach einem der vorhergehenden Ansprüche, **dadurch gekennzeichnet, dass** sie ein Mittel zum Ändern und/oder Erstellen von Gesetzen umfasst, das es dem Benutzer ermöglicht, das vordefinierte Gesetz (L) zu ändern und/oder ein neues Gesetz zu erstellen, das das vordefinierte Gesetz (L) ersetzen soll, die Einschlafhilfevorrichtung vorzugsweise auch ein Fernkommunikationsmodul umfasst, das dazu ausgelegt ist, mit einem Fernbedienungsterminal (4) zu kommunizieren, das die Mittel zum Ändern und/oder Erstellen von Gesetzen enthält, wobei die Kommunikation vorzugsweise eine drahtlose Kommunikation ist.

8. - Einschlafhilfevorrichtung nach dem vorhergehenden Anspruch und nach Anspruch 6, **dadurch gekennzeichnet, dass** das Mittel zum Ändern und/oder Erstellen von Gesetzen es dem Benutzer ermöglicht, die vorbestimmte Ausschaltdauer (dₑ) zu ändern.

9. - Einschlafhilfevorrichtung nach einem der Ansprüche 7 oder 8 und nach Anspruch 4 und/oder 5, **dadurch gekennzeichnet, dass** das Mittel zum Ändern und/oder Erzeugen von Gesetzen es dem Benutzer ermöglicht, den maximalen Leistungswert (Pₘₐₓ) und/oder den Zeitpunkt, zu dem der maximale Leistungswert (Pₘₐₓ) erreicht wird, zu ändern.

10. -Einschlafhilfevorrichtung nach einem der vorhergehenden Ansprüche, **dadurch gekennzeichnet, dass** sie ein Gehäuse (1) umfasst, in dessen Innerem die Lichtquelle (3) angeordnet ist, wobei das Gehäuse (1) eine im Wesentlichen zylindrische Form hat.

11. -Einschlafhilfevorrichtung nach einem der vorhergehenden Ansprüche, **dadurch gekennzeichnet, dass** sie einen Diffusor (2) umfasst, der so gestaltet ist, dass er die Streuung des von der Lichtquelle (3) ausgestrahlten Lichts optimiert, wobei der Diffusor (2) vorzugsweise eine lichtdurchlässige Scheibe umfasst, wobei der Diffusor (2) vorzugsweise eine der Lichtquelle (3) zugewandte Innenseite (21) und eine gegenüberliegende Außenseite (22) umfasst, wobei die Innenseite (21) einen glatteren Oberflächenzustand als der Oberflächenzustand der Außenseite (22) aufweist, wobei der Diffusor (2) vorteilhafterweise aus einem Material hergestellt ist, das mindestens Polycarbonat und ein streuendes Produkt umfasst.

12. -Einschlafhilfevorrichtung nach Anspruch 10 und 11, **dadurch gekennzeichnet, dass** das Gehäuse (1) ein Diffusorfach (15), das seinerseits den Diffusor (2) umfasst, sowie ein Versorgungskompartiment (14) umfasst, das dazu ausgelegt ist, die Lichtquelle (3) mit Energie zu versorgen, wobei das Versorgungskompartiment (14) und das Diffusorfach (15) koaxial mit der zwischen ihnen angeordneten Lichtquelle (3) sind.

13. -Einschlafhilfesystem für einen Benutzers, das eine Vorrichtung nach einem der Ansprüche 7 bis 9 sowie ein Fernbedienungsterminal (4) umfasst, das die Mittel zum Ändern und/oder Erstellen von Gesetzen enthält, wobei das Fernbedienungsterminal (4) so beschaffen ist, dass es mit einem Fernkommunikationsmodul der Vorrichtung kommunizieren kann.

## Claims

1. - A device for inducing sleep in a user, comprising a light source (3) designed to emit a substantially monochromatic red light favouring the falling asleep of the user, said device comprising a control unit designed to automatically control said light source (3) in accordance with a predefined law (L), said device being **characterized in that** according to said predefined law (L), the light power (P) emitted by said light source (3) is progressively decreasing over time (t) so as to evolve automatically between a maximum power value (Pₘₐₓ) and a minimum power value, the light power emitted by said light source (3) at one moment (T) being always higher than or equal to the light power emitted at a later moment (T+1), for the whole duration of use of the sleep-inducing device, said light power never increases after having reached said maximum power value (Pₘₐₓ).

2. - The sleep-inducing device according to the previous claim, **characterized in that** said minimum power value is a substantially null value.

3. - The sleep-inducing device according to one of the preceding claims, **characterized in that** said predefined law (L) is designed so that said light power (P) decreases substantially continuously as a function of time (t) between said maximum power value (Pₘₐₓ) and said minimum power value.

4. - The sleep-inducing device according to one of the preceding claims, **characterized in that** said maximum light power value (Pₘₐₓ) is higher than 600 lux and is advantageously substantially equal to 885 lux, said light source preferably comprising at least one light-emitting diode (LED).

5. - The sleep-inducing device according to one of the preceding claims, **characterized in that** said maximum power value (Pₘₐₓ) is reached when said device is powered on.

6. - The sleep-inducing device according to one of the preceding claims, **characterized in that** said predefined law (L) is designed so that said light source (3) reaches said minimum power value from said maximum power value (Pₘₐₓ) after a predetermined power-off duration (dₑ) has elapsed, said predetermined power-off duration (dₑ) being preferably comprised between 15 and 40 minutes and is more preferably substantially equal to 30 minutes, the sleep-inducing device preferentially further comprising a timer designed to automatically count said predetermined power-off duration (dₑ) from the power-on of said device (t₀), the sleep-inducing device advantageously further comprising a power-off means designed to automatically power said device off at the end of said predetermined power-off duration (dₑ).

7. - The sleep-inducing device according to one of the preceding claims, **characterized in that** it comprises a law modification and/or creation means allowing the user to modify said predefined law (L) and/or to create a new law intended to replace said predefined law (L), the sleep-inducing device preferably further comprising a remote communication module designed to communicate with a remote control terminal (4) that integrates said law modification and/or creation means, said communication being preferentially a wireless communication.

8. - The sleep-inducing device according to the preceding claim and to claim 6, **characterized in that** said law modification and/or creation means allows the user to modify said predetermined power-off duration (dₑ).

9. - The sleep-inducing device according to one of claims 7 or 8 and according to claim 4 and/or 5, **characterized in that** said law modification and/or creation means allows the user to modify the maximum power value (Pₘₐₓ) and/or the moment when said maximum power value (Pmax) is reached.

10. -The sleep-inducing device according to one of the preceding claims, **characterized in that** it comprises a box (1) inside which is arranged said light source (3), said box (1) being of substantially cylindrical shape.

11. -The sleep-inducing device according to one of the preceding claims, **characterized in that** it comprises a diffuser (2) designed to optimize the diffusion of the light emitted by said light source (3), said diffuser (2) preferably comprising a translucent disk, said diffuser (2) preferentially comprising an inner face (21) facing said light source (3) and an opposite, outer face (22), said inner face (21) having a smoother state of surface than the state of surface of said outer surface (22), said diffuser (2) being advantageously made of a material comprising at least polycarbonate and a diffusing product.

12. -The sleep-inducing device according to claims 10 and 11, **characterized in that** said box (1) comprises a diffusion compartment (15) itself comprising said diffuser (2), as well as a power supply compartment (14) designed to supply said light source (3) with power, said power supply compartment (14) and diffusion compartment (15) being coaxial with said light source (3) arranged between each other.

13. -A system for inducing sleep in a user, comprising a device according to any one of claims 7 to 9, as well as a remote control terminal (4) that integrates said law modification and/or creation means, said remote control terminal (4) being designed to communicate with a remote communication module of said device.
